# EUROPEAN PATENT SPECIFICATION

(11) **EP 2 497 517 B1**
(45) Date of publication and mention of the grant of the patent: **05.05.2021**
(21) Application number: 12157617.7
(22) Date of filing: 01.03.2012
(51) Int. Cl.: A61M 25/00, A61M 25/02, A61M 31/00, A61M 5/00, A61M 5/42

(54) **Biomedical device for the injection of contrast means for the execution of fistulography and urethrography**
Biomedizinische Vorrichtung für die Injektion von Kontrastmitteln für die Durchführung einer Fistulografie und Urethrografie
Dispositif biomédical pour l'injection de moyens de contraste pour l'exécution de fistulographie et d'urétrographie

(30) Priority: 03.03.2011 IT MO20110049
(43) Date of publication of application: 12.09.2012
(73) Proprietor: SIDAM S.r.l., 41037 Mirandola (MO) (IT)
(72) Inventor: Azzolini, Graziano, 41032 Cavezzo (MO) (IT)
(74) Representative: Brunacci, Marco

(56) References cited:
- EP-A1- 0 019 199
- FR-A- 440 948
- FR-A1- 2 578 427
- US-A- 1 991 278
- US-A- 2 771 072
- US-A- 3 048 175
- US-A- 4 776 848

## Description

The present invention is defined in the appended claims and relates to a biomedical device for the injection of contrast means, particularly for the execution of fistulography and urethrography.

In the medical field, the execution has been known for some time of an X-ray study of the fistula tract, of the male urethra and of intestinal channels, etc... by means of the use of contrast means.

This technique consists in filling the lumen and any ramifications or loculi of the fistula tract or of the urethra being examined with an iodinated contrast means, in a complete and uniform way.

Obviously, to perform this type of examination, a high injection pressure must be maintained so as to reach all the parts of the cavity to be explored, which often have relaxed walls or are placed downstream of stenotic tracts.

More in particular, to carry out this type of examination using contrast means, catheters are generally used, the extremity of which is positioned inside the organ to be inspected, so as to convey the contrast means inside the required organ.

To ensure the correct positioning on the patient during the injection of the contrast means and to avoid the conformation of the examined parts causing the reflux of the contrast means used, the devices of known type have relative swellings, of the type of inflated balloons, rubber cones or metal screw cones, suitable for obstructing the cutaneous orifice of the fistulas or the urethra meatus.

These devices of known type have some drawbacks.

In fact, the use of these known devices is considerably painful for the patient due to their deep insertion and the presence of the aforementioned swellings which touch the inner walls of the cutaneous orifice of the fistula or the urethra. Another drawback of the known devices consists in the fact that these swellings do not ensure seal against the reflux of the contrast means and, therefore, they are not very effective.

Furthermore, these known devices are not at all practical for the operator who uses them, require long application times and fairly often cause the movement of the catheter, with consequent escape of the contrast means. It therefore follows that devices of known type frequently require the repositioning of the catheter itself, with considerable discomfort for the patient.

These known devices are also dangerous for the operator, who can be contaminated by the contrast means coming out of the catheter following the shift of the latter from the correct work position.

Yet another drawback of known devices consists in the fact that they require long application and use times, hence considerable discomfort for the patient and a prolonged occupation of the relative diagnostic rooms and, therefore, a reduced efficiency of the health system.

EP0019199 discloses an apparatus for injection of radio-opaque fluid in a male urethra comprising: a cylinder which is designed to receive and hold the penis and in which a subatmospheric pressure can be produced; a connecting tube for feeding the fluid into the interior of the cylinder, at which free end is fixed a "gum olive", placed inside the cylinder, and fixed at the free of the tube. US2771072 discloses an injection and insufflation cannula for hysterography in gynaecological therapy.

The main object of the present invention is to provide a biomedical device for the injection of contrast means as defined in the appended claims and which allows, compared to devices of known type, considerably reducing the pain caused to patients by their application and their use.

Within this aim, one object of the present invention is to provide a device that is easy and fast to apply.

One object of the present invention is to provide a device which is less invasive for the patient with respect to the devices of known type.

Another object of the present invention is to provide a device that permits successfully preventing the reflux of the contrast means injected into the patient. Yet another object of the present invention is to provide a device that allows avoiding the contamination of the operator, as well as of the room and of the equipment used, by the contrast means.

Not the last object of the present invention is to considerably reduce the time required for the application and the removal of devices of known type, thereby reducing the time of occupation of the relative diagnostic rooms and the waiting time for the patients and, consequently, allowing the performance of a larger number of examinations as well as the optimisation of the use of the medical facilities.

Another object of the present invention is to provide a biomedical device for the injection of contrast means that allows overcoming the mentioned drawbacks of the state of the art within the ambit of a simple, rational, easy and effective to use as well as low cost solution.

The above mentioned objects are achieved by the present biomedical device for the injection of contrast means, particularly for the execution of fistulography and urethrography, the biomedical device comprises:
- a hollow body which defines at least a chamber having an opening turned to the outside and intended to be positioned in correspondence to the body of a patient and a suction mouth connectable to suction means suitable for defining a vacuum in said chamber;
- at least a cannula associated with said body, which crosses said chamber and which has at least an inlet gap and at least an outlet gap of a contrast means.

Other characteristics and advantages of the present invention will become more evident from the description of a preferred, but not sole, embodiment of a biomedical device for the injection of contrast means, illustrated purely as an example but not limited to the annexed drawings in which:
Figure 1 is an axonometric view of a device according to the invention in a first embodiment;
Figure 2 is a section view of the device of Figure 1 along the track plane II-II;
Figure 3 is an axonometric view of a device according to the invention in a second embodiment;
Figure 4 is a section view of the device of Figure 3 along the track plane IV-IV;

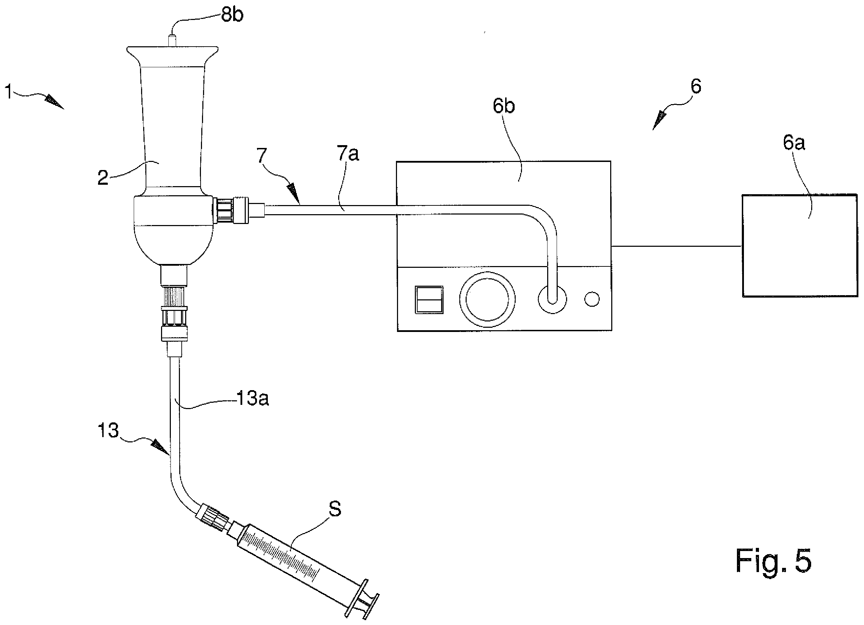

Figure 5 is a schematic view of the device of Figure 1 connected to suction means.

With particular reference to such figures, globally indicated by 1 is a biomedical device for the injection of contrast means, particularly for the execution of fistulography and urethrography.

The device 1 comprises at least one hollow body 2 defining at least one chamber 3 having an opening 4 turned towards the outside and intended to be positioned in correspondence to the body of a patient, and a suction mouth 5 connectable to suction means 6 suitable for defining a vacuum inside the chamber 3.

Suitably, the device 1 comprises at least one junction member 7 of the body 2 to the suction means 6.

More in detail, the junction member 7 comprises at least a tube 7a of the flexible type. Along the tube 7a is arranged a hydrophobic filter, not visible in detail in the illustrations, suitable for preventing the bacterial contamination of the suction means 6 by biological fluids coming from the patient.

More in particular, the suction means 6 comprise a vacuum pump 6a suitable for recalling air so as to apply a vacuum inside the chamber 3, and adjustment means 6b for adjusting the vacuum connected to the pump 6a, suitable for allowing the adjustment of the vacuum exercised by the pump itself.

The device 1 then comprises at least one cannula 8 associated with the body 2, which crosses the chamber 3 and has at least one inlet gap 8a and at least one outlet gap 8b of a contrast means, where the outlet gap 8b can be positioned inside the organ to be inspected.

Advantageously, the cannula 8 is associated sliding with the body 2.

The relative movement of the cannula 8 and of the body 2 allows these to be perfectly positioned with respect to the patient's body and therefore avoids the occurrence of traumatic events on the patient him/herself.

More in particular, the cannula 8 is mobile with respect to the body 2 along a direction which substantially coincides with the longitudinal extension of the cannula itself.

The cannula 8 is isolated from the chamber 3 and its inlet gap 8a and outlet gap 8b are placed in communication with the outside and do not therefore communicate with the chamber itself.

In the preferred, but not only embodiments, shown in the figures, the cannula 8 is made up of an intermediate member 9, which is associated with the body 2 by means of sealing means 10 and wherein is defined a transit duct 9a, of a tubular member 11 fitted inside the intermediate member 9 coaxially to the duct 9a, and of a coupling member 12 associated with the intermediate member 9 on the opposite side of the tubular member 11 and this too defining a transit channel 12a communicating with the duct 9a.

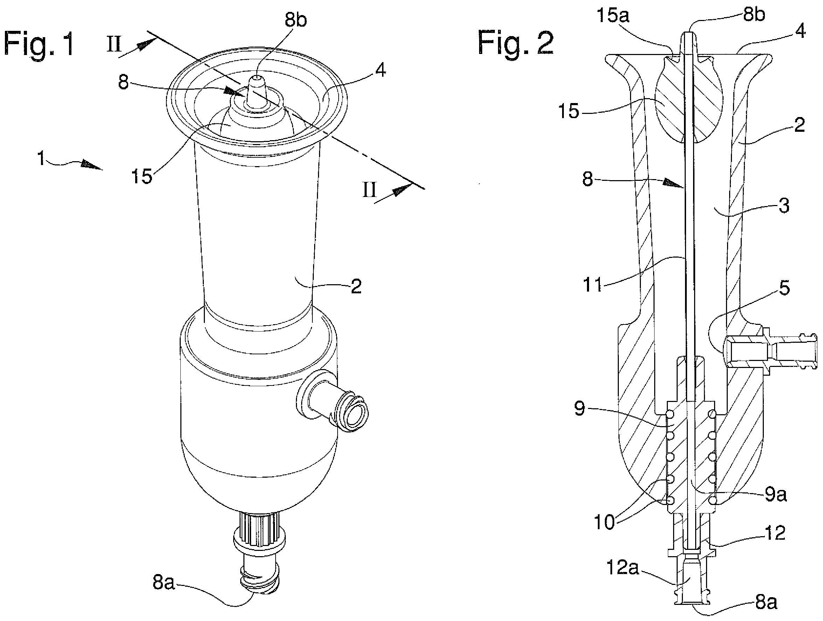

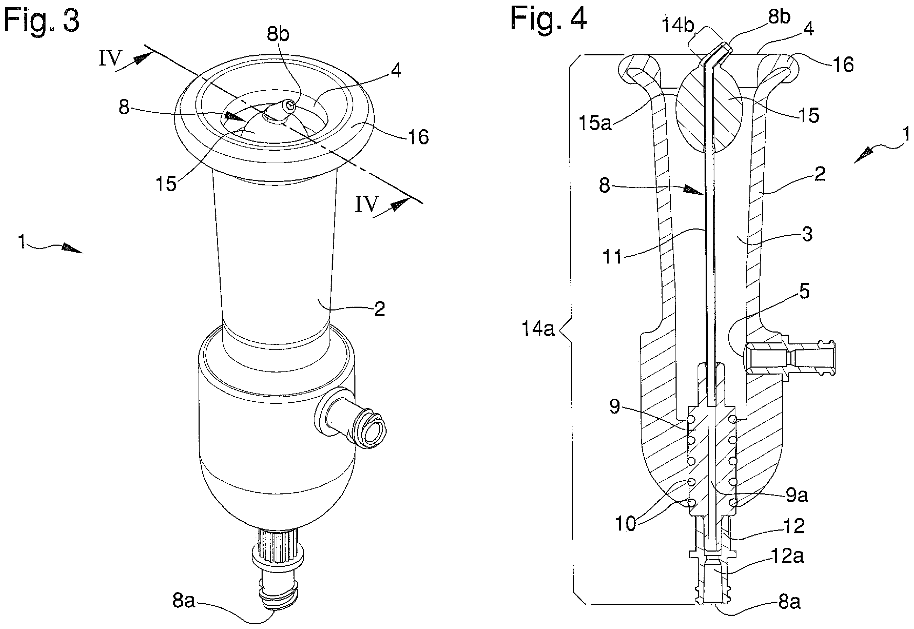

Preferably, the intermediate member 9 is substantially fitted to measure inside a housing seat defined on the body 2 in such a way as to be mobile sliding inside it. As can be seen in the figures 2 and 4, the sealing means 10 comprise a plurality of elastic rings placed between the intermediate member 9 and the body 2 in such a way as to ensure seal quite apart from their reciprocal position. It cannot however be ruled out that two or more of the aforementioned components making up the cannula 8 are defined integrally the one with the other or in a single piece with the body 2.

Alternative embodiments cannot however be ruled out wherein the cannula 8 is associated integral with the body 2.

The inlet gap 8a is associable with an intake line S of the contrast means such as, for example, a syringe.

Suitably, the device 1 comprises at least a connection member 13 for connecting the cannula 8 to the intake line S. The connection member 13 comprises, e.g., a flexible hose 13a communicating with the inlet gap 8a. The connection member 13 also comprises a unidirectional valve, not visible in detail in the illustrations, suitable for preventing the return of the injected contrast means. Advantageously, the outlet gap 8b of the cannula 8 protrudes from the opening 4 to allow its introduction inside the organ to be inspected.

Preferably, the section of the chamber 3 is variable and growing towards the opening 4.

More in particular, at least one portion of the inner walls of the body 2 which delimit the chamber 3 is diverging towards the opening 4. The volume of the chamber 3 therefore increases upon moving towards the opening 4.

In the embodiments shown in the illustrations, the chamber 3 has a substantially circular section.

As shown in the attached illustrations and in particular in figures 2 and 4, the cannula 8 comprises at least a rectilinear section substantially coaxial with the chamber 3.

In the first embodiment of the device 1 shown in the figures 1 and 2, particularly suitable for executing urethrography, the cannula 8 is rectilinear and entirely coaxial with the chamber 3.

In the second embodiment of the device 1 particularly suitably for executing fistulography, the cannula 8 can be substantially rectilinear and coaxial with the chamber 3 or, as shown in the figures 3 and 4, can comprise a first section 14a substantially coaxial with the chamber 3 and a second section 14b inclined with respect to the first section 14a. The first section 14a comprises the inlet gap 8a and the second section 14b comprises the outlet gap 8b. The inclination between the first and the second sections 14a and 14b can vary according to the particular geometry of the part to undergo examination.

Preferably, the inclination between the first and the second parts 14a and 14b is between 40° and 50°.

Suitably, the inner walls of the body 2 which delimit the opening 4 are tapered outwards so as to define a stable support base for the patient's body.

Preferably, the device 1 comprises at least a sealing member 16 associated with the body 2 in correspondence to the edge thereof which delimits the opening 4. More in particular, the sealing member 16 is made of an elastically deformable material, e.g., a spongy material, so as to adapt to any lack of uniformity of the patient's body, such as scars or the like, for the purpose of maintaining the seal of the vacuum exercised inside the chamber 3. The use of the sealing member 16 is particularly important in the embodiment suitable for the execution of fistulography, shown in the figures 3 and 4.

Advantageously, the cannula 8 comprises locator means 15 suitable for externally resting on the patient's body to prevent the further insertion of the outlet gap 8b inside the organ to be examined.

More in particular, the locator means 15 define a swelling along the cannula 8. Such swelling therefore defines a locator surface 15a intended to contact the patient's body.

In the first embodiment of figures 1 and 2, particularly suitable for the execution of urethrography, the locator surface 15a is substantially concave, so as to rest on the outer surface of the gland.

In the second embodiment shown in the figures 3 and 4, the locator surface 15a is instead substantially convex.

Different embodiments of the locator means 15 cannot however be ruled out both for the first and the second embodiments.

Suitably, the locator means 15 can be integrally defined with the tubular member 11, or can be made separately and fitted on the tubular member itself. In the event of the locator means 15 being distinct from the tubular member 11, the outlet gap 8b of the cannula 8 can be defined by the locator means themselves.

The operation of the device of the invention is the following. First of all the device 1 is connected to the suction means 6 and in particular to the adjustment means 6b.

Afterwards the outlet gap 8b of the cannula 8 is inserted inside the urethra or the fistula to be examined, making this pass through an orifice made surgically or already existing, such as the urethra, until the locator surface 15a contacts the patient's body. In the particular case in which a urethrography has to be performed, the outlet gap 8b is inserted until the locator surface 15a contacts the patient's gland.

The section of the cannula 8 which is inserted inside the patient is therefore only that positioned between the outlet gap 8b and the locator surface 15a. Subsequently, the edge of the body 2 delimiting the opening 4 is moved into contact with the patient's body so as to close the opening itself.

By exploiting the axial sliding of the cannula 8 with respect to the body 2 the best positioning of these two members is sought with respect to the patient's body, in such a way as to prevent injuries for the patient him/herself and at the same time ensure the correct resting of the opening 4 on the patient's body, a condition required to apply the vacuum inside the chamber 3.

In the event of its being intended to use the device 1 for the execution of a fistulography, the correct positioning of the opening 4 on the patient's body, and therefore the seal of the chamber 3, is ensured by the sealing member 16 which offsets, by deforming itself, the presence of any wounds or discontinuities on the patient's skin.

At this point, the chamber 3 is isolated from the outside, and so by operating the pump 6a, a vacuum is created inside the chamber 3 which ensures the adhesion of the body 2 to the gland or to the tissue surrounding the fistula.

By means of the adjustment means 6b, the intensity can be changed of the vacuum applied in the chamber 3 adapting it to the specific area treated and thus causing the device 1 to adhere to the patient's body in a non-traumatic and painless way.

The presence of the water-repellent filter arranged along the tube 7a of the junction member 7 protects the suction means 6, and more in general, the hydraulic circuit connected to them, from pollution by the patient's biological liquids.

After fitting therefore the device 1 to the patient's body, the next step is to inject the contrast means into the patient him/herself.

More in particular, the syringe S containing the contrast means is fitted inside the connection member 13 and, therefore, placed in communication with the cannula 8.

By pressing the piston of the syringe S, the contrast means crosses the connection member 13 and the cannula 8 until it comes out of the outlet gap 8b of the cannula itself.

The unidirectional valve of the connection member 13 prevents the return of the fluid and therefore allows making periodical injections, alternated with intervals, without forcing the operator to continually press the syringe, and without any risk of the contrast means coming out.

Once the introduction of the contrast means has terminated, by means of the adjustment means 6b the vacuum exercised inside the chamber 3 is interrupted, after which the device 1 can be easily removed from the patient's body, extracting the outlet gap 8b.

It has in practice been ascertained how the described invention achieves the proposed objects and in particular the fact is underlined that the device according to the invention can be fitted to a patient in an easy and completely non-traumatic way.

In fact, the device according to the invention does not contemplate any protuberance which has to be inserted inside the organ to be examined to ensure its seal, which is provided by the vacuum applied inside the chamber of the device itself.

The use of the device of the invention is therefore completely painless for the patient, inasmuch as the cannula through which the contrast means is injected is only introduced by a minimum section such as to ensure the introduction of the contrast means itself. Furthermore, the geometry of the locator means is such as to be completely non-traumatic for the patient.

Again, the device according to the invention allows protecting the operators and the surrounding environment from any possible contamination by the injected contrast means.

The device according to the invention therefore allows considerably reducing, with respect to devices of known type, the time required to perform an investigation by means of contrast means and the waiting time, thus increasing the productivity of the health system.

## Claims

1. Biomedical device (1) for the injection of contrast means for the execution of fistulography and urethrography, comprising:
- a hollow body (2) which defines at least a chamber (3) having an opening (4) turned to the outside and intended to be restingly positioned on the body of a patient and a suction mouth (5); and
- at least a cannula (8) associated with said body (2), which crosses said chamber (3) and which has at least an inlet gap (8a) and at least an outlet gap (8b) of a contrast means, said outlet gap (8b) being positionable inside the organ to be inspected;
wherein the outlet gap (8b) protrudes from said opening (4) so that it can be introduced inside said organ, the cannula (8) being isolated from the chambre (3) and its inlet gap (8a) and its outlet gap (8b) being placed in communication with the outside and do not therefore communicate with the chamber itself, and said cannula (8) comprises locator means (15), defining a swelling, and placed along the cannula (8) so as to define an insertion section of the cannula (8) between the outlet gap (8b) and the locator means (15), the latter being able to externally rest on said body so as to prevent a further insertion of the outlet gap (8b) inside said organ, and wherein said locator means (15) define at least a locator surface (15a) intended to contact the patient's body, **characterized by** the fact that said cannula (8) is associated sliding with said body (2) and said locator surface (15a) is arranged in proximity to said outlet gap (8b).

2. Device (1) according to claim 1, **characterised by** the fact that at least a portion of the inner walls of said body (2) which delimit said chamber (3) is diverging towards said opening (4).

3. Device (1) according to one or more of the preceding claims, **characterised by** the fact that the inner walls of said body (2) which delimit said opening (4) are tapered outwards.

4. Device (1) according to one or more of the preceding claims, **characterised by** the fact that it comprises at least a sealing element (16) associated with said body (2) in correspondence to its edge delimiting said opening (4), said sealing element (16) being intended to rest on the patient's body to keep said chamber (3) isolated from the outside.

5. Device (1) according to one or more of the preceding claims, **characterised by** the fact that said chamber (3) has a substantially circular section.

6. Device (1) according to one or more of the preceding claims, **characterised by** the fact that at least a section of said cannula (8) is substantially coaxial to said chamber (3).

7. Device (1) according to one or more of the preceding claims, **characterised by** the fact that said cannula (8) has at least a first section (14a), comprising said inlet gap (8a), and at least a second section (14b), comprising said outlet gap (8b), inclined with respect to said first section (8a).

8. Device (1) according to claim1, **characterised by** the fact that said locator surface (15a) is concave.

9. Device (1) according to one or more of the preceding claims, **characterised by** the fact that said cannula (8) is isolated from said chamber (3).

## Patentansprüche

1. Biomedizinische Vorrichtung (1) zur Injektion von Kontrastmittel für die Durchführung von Fistulographie und Urethrographie, umfassend:
- einen Hohlkörper (2), der mindestens eine Kammer (3) mit einer zur Außenseite gerichteten Öffnung (4) definiert, die dazu bestimmt ist, auf dem Körper eines Patienten aufzuliegen, und eine Saugmündung (5); und
- mindestens eine mit dem Körper (2) verbundene Kanüle (8), die die Kammer (3) durchquert und die mindestens einen Einlassspalt (8a) und mindestens einen Auslassspalt (8b) eines Kontrastmittels aufweist, wobei der Auslassspalt (8b) innerhalb des zu untersuchenden Organs positionierbar ist;
wobei der Auslassspalt (8b) aus der Öffnung (4) herausragt, so dass er in das Organ eingeführt werden kann, wobei die Kanüle (8) von der Kammer (3) isoliert ist und ihr Einlassspalt (8a) und ihr Auslassspalt (8b) in Verbindung mit der Außenseite stehen und daher nicht mit der Kammer selbst in Verbindung stehen, und die Kanüle (8) Lokalisierungsmittel (15) umfasst, die eine Wölbung definieren und entlang der Kanüle (8) so angeordnet sind, dass sie einen Einführungsabschnitt der Kanüle (8) zwischen dem Auslassspalt (8b) und den Lokalisierungsmitteln (15) definieren, letztere in der Lage sind, von außen auf dem Körper aufzuliegen, um ein weiteres Einführen des Auslassspalts (8b) in das Organs zu verhindern, und wobei die Lokalisierungsmittel (15) mindestens eine Lokalisierungsfläche (15a) definieren, die dazu bestimmt ist, den Körper des Patienten zu berühren, **dadurch gekennzeichnet, dass** die Kanüle (8) gleitend mit dem Körper (2) verbunden ist und die Lokalisierungsfläche (15a) in der Nähe des Auslassspalts (8b) angeordnet ist.

2. Vorrichtung (1) nach Anspruch 1, **dadurch gekennzeichnet, dass** zumindest ein Teil der Innenwände des Körpers (2), die die Kammer (3) begrenzen, zur Öffnung (4) hin divergiert.

3. Vorrichtung (1) nach einem oder mehreren der vorhergehenden Ansprüche, **dadurch gekennzeichnet, dass** die Innenwände des Körpers (2), die die Öffnung (4) begrenzen, nach außen hin verjüngt sind.

4. Vorrichtung (1) nach einem oder mehreren der vorhergehenden Ansprüche, **dadurch gekennzeichnet, dass** sie mindestens ein Dichtungselement (16) umfasst, das dem Körper (2) in Übereinstimmung mit seinem Rand, der die Öffnung (4) begrenzt, zugeordnet ist, wobei das Dichtungselement (16) dazu bestimmt ist, auf dem Körper des Patienten aufzuliegen, um die Kammer (3) von der Außenseite isoliert zu halten.

5. Vorrichtung (1) nach einem oder mehreren der vorhergehenden Ansprüche, **dadurch gekennzeichnet, dass** die Kammer (3) einen im Wesentlichen kreisförmigen Querschnitt aufweist.

6. Vorrichtung (1) nach einem oder mehreren der vorhergehenden Ansprüche, **dadurch gekennzeichnet, dass** zumindest ein Abschnitt der Kanüle (8) im Wesentlichen koaxial zur Kammer (3) ist.

7. Vorrichtung (1) nach einem oder mehreren der vorhergehenden Ansprüche, **dadurch gekennzeichnet, dass** die Kanüle (8) mindestens einen ersten Abschnitt (14a), der den Einlassspalt (8a) umfasst, und mindestens einen zweiten Abschnitt (14b), der den Auslassspalt (8b) umfasst, aufweist, der gegenüber dem ersten Abschnitt (8a) geneigt ist.

8. Vorrichtung (1) nach Anspruch 1, **dadurch gekennzeichnet, dass** die Lokalisierungsfläche (15a) konkav ist.

9. Vorrichtung (1) nach einem oder mehreren der vorhergehenden Ansprüche, **dadurch gekennzeichnet, dass** die Kanüle (8) von der Kammer (3) isoliert ist.

## Revendications

1. Dispositif biomédical (1) pour l'injection de moyens de contraste pour l'exécution d'une fistulographie et d'une urétrographie, comprenant :
- un corps creux (2) qui définit au moins une chambre (3) présentant une ouverture (4) tournée vers l'extérieur et destinée à être positionnée en appui sur le corps d'un patient et une bouche d'aspiration (5) ; et
- au moins une canule (8) associée audit corps (2), qui croise ladite chambre (3) et qui comporte au moins un espace d'entrée (8a) et au moins un espace de sortie (8b) d'un moyen de contraste, ledit espace de sortie (8b) pouvant être positionné à l'intérieur de l'organe à inspecter ;
dans lequel l'espace de sortie (8b) fait saillie depuis ladite ouverture (4) de telle sorte qu'il peut être introduit à l'intérieur dudit organe, la canule (8) étant isolée de la chambre (3) et son espace d'entrée (8a) et son espace de sortie (8b) étant placés en communication avec l'extérieur et ne communiquant par conséquent pas avec la chambre elle-même, et ladite canule (8) comprend un moyen localisateur (15), définissant un gonflement, et placé le long de la canule (8) de manière à définir une section d'intersection de la canule (8) entre l'espace de sortie (8b) et le moyen localisateur (15), ce dernier pouvant s'appuyer extérieurement sur ledit corps de manière à empêcher une insertion supplémentaire de l'espace de sortie (8b) à l'intérieur dudit organe, et dans lequel ledit moyen localisateur (15) définit au moins une surface de localisateur (15a) destinée à entrer en contact avec le corps du patient, **caractérisé en ce que** ladite canule (8) est associée de manière coulissante audit corps (2) et ladite surface de localisateur (15a) est agencée à proximité dudit espace de sortie (8b).

2. Dispositif (1) selon la revendication 1, **caractérisé en ce qu'**au moins une partie des parois intérieures dudit corps (2) qui délimitent ladite chambre (3) diverge vers ladite ouverture (4).

3. Dispositif (1) selon une ou plusieurs des revendications précédentes, **caractérisé en ce que** les parois intérieures dudit corps (2) qui délimitent ladite ouverture (4) se resserrent vers l'extérieur.

4. Dispositif (1) selon une ou plusieurs des revendications précédentes, **caractérisé en ce qu'**il comprend au moins un élément d'étanchéité (16) associé audit corps (2) en correspondance avec son bord délimitant ladite ouverture (4), ledit élément d'étanchéité (16) étant destiné à s'appuyer sur le corps du patient pour maintenir ladite chambre (3) isolée de l'extérieur.

5. Dispositif (1) selon une ou plusieurs des revendications précédentes, **caractérisé en ce que** ladite chambre (3) présente une section sensiblement circulaire.

6. Dispositif (1) selon une ou plusieurs des revendications précédentes, **caractérisé en ce qu'**au moins une section de ladite canule (8) est sensiblement coaxiale avec ladite chambre (3).

7. Dispositif (1) selon une ou plusieurs des revendications précédentes, **caractérisé en ce que** ladite canule (8) présente au moins une première section (14a), comprenant ledit espace d'entrée (8a), et au moins une seconde section (14b), comprenant ledit espace de sortie (8b), inclinée par rapport à ladite première section (8a).

8. Dispositif (1) selon la revendication 1, **caractérisé en ce que** ladite surface de localisateur (15a) est concave.

9. Dispositif (1) selon une ou plusieurs des revendications précédentes, **caractérisé en ce que** ladite canule (8) est isolée de ladite chambre (3).
